(19) Europäisches Patentamt
European Patent Office
Office européen des brevets

(11) **EP 2 611 376 B1**

(12) **FASCICULE DE BREVET EUROPEEN**

(45) Date de publication et mention
de la délivrance du brevet:
**31.08.2016 Bulletin 2016/35**

(21) Numéro de dépôt: **10752760.8**

(22) Date de dépôt: **02.09.2010**

(51) Int Cl.:
*A61B 17/80* (2006.01)     *A61B 17/86* (2006.01)

(86) Numéro de dépôt international:
**PCT/EP2010/062899**

(87) Numéro de publication internationale:
**WO 2012/028192 (08.03.2012 Gazette 2012/10)**

(54) **DISPOSITIF D'OSTÉOSYNTHÈSE**

OSTEOSYNTHESEVORRICHTUNG

OSTEOSYNTHESIS DEVICE

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(43) Date de publication de la demande:
**10.07.2013 Bulletin 2013/28**

(73) Titulaire: **Neosteo**
**44100 Nantes (FR)**

(72) Inventeur: **DEROUET, Guillaume**
**44420 La Turballe (FR)**

(74) Mandataire: **Godineau, Valérie et al**
**Ipsilon**
**3, rue Edouard Nignon**
**44300 Nantes (FR)**

(56) Documents cités:
**WO-A1-2010/073103     WO-A2-2008/087360**
**US-A1- 2007 088 360     US-A1- 2008 188 899**
**US-A1- 2008 306 550**

**Description**

Le domaine de l'invention est celui des techniques d'ostéosynthèse.

Plus précisément, l'invention concerne un dispositif d'ostéosynthèse du type comprenant une plaque destinée à être appliquée sur un os et une tige filetée destinée à assurer le maintien de fragments osseux par rapport à la plaque.

Les techniques d'ostéosynthèse sont mises en oeuvre pour opérer la consolidation osseuse de certaines fractures nécessitant le montage, sur les fragments osseux, d'un dispositif conçu pour stabiliser et empêcher une mobilité inter-fragmentaire excessive.

Selon une technique classique, un dispositif d'ostéosynthèse comprend :

- un support ou une plaque présentant au moins un taraudage fileté, cette plaque étant destinée à être appliquée de façon adaptée sur les fragments osseux avec la meilleure congruence possible ;
- au moins une tige présentant à sa base un premier filetage destiné à assurer l'ancrage de la tige dans l'os et présentant un deuxième filetage destiné à coopérer avec un taraudage fileté de la plaque de façon à appliquer, après vissage complet de la tige, un serrage de la plaque contre l'os (ou les fragments osseux).

Les dimensions des différents éléments constituant le dispositif d'ostéosynthèse sont relativement réduites de façon à limiter le traumatisme d'implantation ; en particulier, le diamètre de la tige et la hauteur des filets de celle-ci sont de petites tailles.

Aussi, avec de tels dispositifs, il est fréquent, lors de leur pose, de déplorer un défaut de coaxialité entre la tige et le taraudage fileté de la plaque, au risque d'opérer un montage à faux filets, auquel cas, il devient difficile voire impossible d'effectuer un serrage optimal de la tige dans la plaque.

Pour tenter de remédier à cet inconvénient et/ou pour améliorer la congruence de la plaque sur l'os, il a été proposé de réaliser des tiges filetées présentant un filetage destiné à coopérer avec un taraudage fileté de la plaque, telles que le fond de filet dudit filetage et la crête dudit filetage présentent tous deux la forme d'un corps tronconique.

Le document US 2008/0306550 A1 décrit un dispositif d'ostéosynthèse conforme au préambule de la revendication 1.

Toutefois, on constate dans la pratique qu'une telle tige engendre plusieurs inconvénients, et notamment :

- le serrage de la tige sur la plaque engendre, dans certains cas, une compression et/ou des déformations telles, que le retrait des tiges filetées après serrage est très difficile, voire impossible ;
- le serrage de la tige filetée sur la plaque engendre un effet de tire-fond qui tend à tirer la plaque vers la tête de la tige et, par conséquent, à trop espacer la plaque de l'os.

L'invention a notamment pour objectif de pallier ces inconvénients de l'art antérieur.

Plus précisément, l'invention a pour objectif de proposer un dispositif d'ostéosynthèse qui permette, après serrage, d'effectuer sans moyen supplémentaire le retrait des tiges filetées vissées à la plaque.

L'invention a également pour objectif de fournir un tel dispositif d'ostéosynthèse qui assure une congruence optimale de la plaque sur l'os.

Ces objectifs, ainsi que d'autres qui apparaîtront par la suite, sont atteints grâce à l'invention qui a pour objet un dispositif d'ostéosynthèse tel que défini dans la revendication 1.

Avec un tel dispositif d'ostéosynthèse, les contraintes de serrage longitudinales de la tige sur la plaque s'exercent de façon telle que la plaque est entraînée progressivement par la tige filetée pénétrant dans l'os jusqu'à appliquer parfaitement la plaque contre l'os et obtenir une congruence optimale.

Selon l'invention, le deuxième filetage de la tige présente un filet dont le diamètre nominal décroît régulièrement de la tête de vissage à la base de la tige, alors que le diamètre de fond du filet est, comme sur une vis standard, constant.

Il en résulte que, lors du serrage de la tige dans le taraudage fileté correspondant de la plaque, on limite considérablement les contraintes radiales de la tige sur le taraudage fileté de la plaque. En particulier, le risque que le serrage de la tige sur la plaque conduise à un grippage est considérablement réduit, en comparaison avec les montages réalisés avec des vis de l'art antérieur. En effet, la distance entre la crête et le fond de filet du deuxième filetage est supérieure ou égale à la distance présentée par les vis usuelles, et elle augmente régulièrement jusqu'à un maximum atteint à l'extrémité de la tête de vissage, ce qui limite les contraintes entre la tige et la plaque au fur et à mesure du serrage.

Ainsi, une tige filetée selon l'invention est moins sujette aux déformations et évite d'engendrer des déformations sur le taraudage fileté correspondant de la plaque. Une telle tige filetée est donc plus facilement retirable, après serrage sur la plaque.

Avantageusement, le deuxième filetage est obtenu par écrêtage d'une vis présentant un diamètre nominal D et un pas P, le fond de filet cylindrique dudit deuxième filetage présentant un diamètre d3 tel que d3 > D - 1,2268 x P.

De cette façon, le diamètre de fond de filet d3 du deuxième filetage est supérieur au diamètre de fond de filet D3 norm d'une vis classique, calculé suivant la norme ISO par la formule D3 norm = D - 1,2268 x P.

Ainsi, la résistance de la tige filetée peut être renforcée.

De plus, l'orifice prévu dans la tête de la tige filetée pour permettre l'introduction d'un outil de vissage, de type à six pans creux par exemple, utilisé pour effectuer le vissage sur la plaque, peut être agrandi. Un outil de vissage plus grand par rapport à l'art antérieur pour cette taille de tige filetée peut alors être utilisé, ce qui en termes de couple permet de faciliter les opérations de vissage et de dévissage.

De façon préférentielle, le diamètre de fond de filet d3 dudit deuxième filetage est tel que d3 ≤ D - 0,8872 x P.

Cette caractéristique induit que le diamètre de fond de filet du deuxième filetage, quoiqu'augmenté par rapport au diamètre D3 norm des vis standard, reste limité de façon à offrir une tolérance de désaxage, au début du mouvement de vissage de la tige filetée dans la plaque, tout en évitant les interférences entre le fond de filet du deuxième filetage et la crête du taraudage fileté.

Selon une solution avantageuse, l'angle de conicité du tronc de cône défini par la crête du deuxième filetage est compris entre 1° et 60°, l'angle de conicité dudit tronc du cône étant préférentiellement de 9°.

Selon une autre caractéristique avantageuse, ledit taraudage fileté présente une crête définissant un deuxième tronc de cône.

Préférentiellement, le premier filetage de la tige filetée présente un premier pas, le deuxième filetage présentant un deuxième pas inférieur ou égal audit premier pas.

Une telle caractéristique contribue à son niveau à optimiser la mise en compression de la plaque sur l'os et à éviter tout effet de tire-fond.

En effet, avec une telle tige filetée, le serrage de la tige avec la plaque s'effectue plus progressivement que l'ancrage de la tige dans l'os.

Selon un mode de réalisation avantageux, ladite tige présente une tête surmontant ledit deuxième filetage, ladite tête étant apte à venir se loger complètement dans ladite plaque et présentant une surface destinée à venir en butée contre une surface de forme complémentaire ménagée dans ladite plaque.

Dans ce cas, ladite surface de ladite tête et ladite surface de forme complémentaire ménagée dans ladite plaque présentent une forme appartenant au groupe suivant :

- tronc de sphère ;
- tronc de cône ;
- annulaire et plate.

Selon une variante avantageuse, ledit premier filetage se décompose en deux parties :

- une partie d'extrémité présentant un filetage cylindrique ;
- une partie intermédiaire s'étendant entre ladite partie d'extrémité et ledit deuxième filetage, ladite partie intermédiaire présentant un filetage conique.

On obtient de cette façon un ancrage optimisé de la tige dans l'os, ceci de par la présence de la partie intermédiaire et de son filetage conique. En outre, une telle première partie (avec le filetage conique de la partie intermédiaire) optimise le fait d'éviter l'effet tire-fond mentionné précédemment lors du serrage de la tige sur la plaque.

D'autres caractéristiques et avantages de l'invention apparaîtront plus clairement à la lecture de la description suivante d'un mode de réalisation préférentiel de l'invention, donné à titre d'exemple illustratif et non limitatif, et des dessins annexés parmi lesquels :

- la figure 1 est une représentation schématique simplifiée d'une tige filetée d'un dispositif d'ostéosynthèse selon l'invention ;
- la figure 2 est une représentation schématique simplifiée d'une plaque d'un dispositif d'ostéosynthèse selon l'invention ;
- la figure 3 est une vue en coupe partielle d'un taraudage fileté d'une plaque d'un dispositif d'ostéosynthèse selon l'invention ;
- la figure 4 est une vue d'une tige filetée vissée dans une plaque d'un dispositif d'ostéosynthèse selon l'invention ;

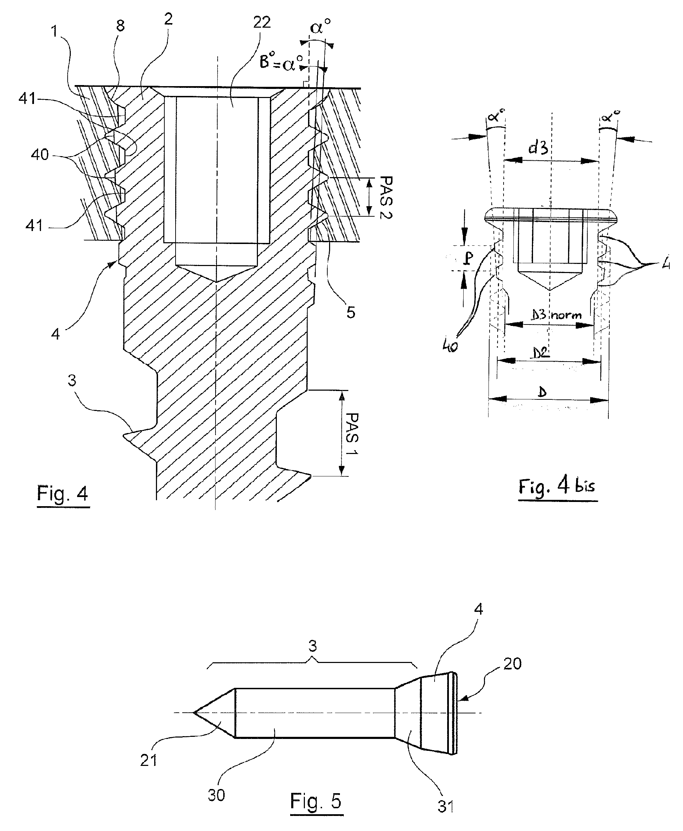

- la figure 4 bis est une vue partielle d'une tige filetée, dans sa partie comprenant le deuxième filetage, selon l'invention ;
- la figure 5 est une vue d'une tige filetée d'un dispositif d'ostéosynthèse selon une variante de réalisation de l'invention.

Tel qu'indiqué précédemment, le principe de l'invention consiste à proposer un dispositif d'ostéosynthèse, comprenant une plaque et au moins une tige filetée, dans lequel la tige filetée présente un filetage de coopération avec la plaque, ce filetage s'étendant à partir d'un corps cylindrique et dont la hauteur de filet décroît en allant vers la base de la tige filetée.

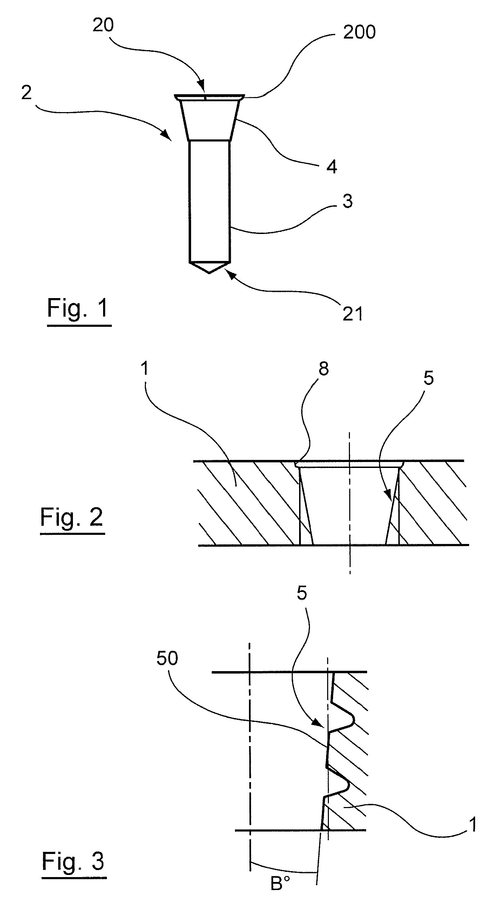

En référence aux figures 1 et 2, un dispositif selon l'invention comprend :

- une plaque 1, présentant au moins un taraudage fileté 5 ;
- au moins une tige filetée 2 présentant une tête de vissage 20 et, à son extrémité opposée à la tête de vissage, une base 21, la tige filetée présentant deux filetages successifs 3, 4 entre la base 21 et la tête de vissage 20.

Sur une portion s'étendant à partir de la base 21 de la tige filetée, un premier filetage 3 est destiné à assurer l'ancrage de la tige dans un os (voire dans un fragment d'os).

Dans une portion de la tige s'étendant à partir de la tête 20, la tige filetée présente un deuxième filetage 4 destiné à coopérer avec un taraudage fileté 5 ménagé dans la plaque 1.

Selon le principe de l'invention et en référence aux figures 4 et 4 bis, le deuxième filetage 4 s'étend radialement, à partir d'un fond de filet dont la surface 41 définit un corps de forme cylindrique, jusqu'à une crête 40 définissant un tronc de cône. En d'autres termes, le fond de filet du deuxième filetage 4 définit un cylindre, tandis que la crête du deuxième filetage 4 définit une enveloppe tronconique.

Un tel filetage est réalisé par exemple par usinage d'un corps cylindrique, de façon à réaliser dans un premier temps un filetage classique (dont la hauteur de crête est constante). Dans un deuxième temps, on procède à une deuxième étape d'usinage du filet préalablement réalisé, de façon à tronquer celui-ci progressivement en vue d'obtenir une crête définissant un tronc de cône dont le diamètre le plus important est présent au voisinage de la tête de vissage.

On obtient donc de cette façon une hauteur de filet qui décroît régulièrement sur la longueur du deuxième filetage, en allant de la tête de vissage en direction de la base de la tige filetée.

On note que l'usinage de la crête du filetage entraîne la formation d'une surface aplatie hélicoïdale (plutôt qu'un bord saillant comme c'est le cas avec les filets classiques).

Sur la figure 4bis, P désigne le pas et D le diamètre nominal, avant écrétage, du deuxième filetage 4 de la tige filetée.

De plus, D3 norm désigne le diamètre de fond de filet d'une vis présentant un diamètre nominal D et un pas P, suivant la norme ISO selon laquelle D3 norm = D - 1,2268 x P.

Enfin, D2 représente le diamètre à flancs de filet et d3 le diamètre de fond de filet du deuxième filetage 4.

Selon un mode de réalisation préféré, le diamètre de fond de filet d3 du deuxième filetage est tel que d3 > D3 norm et d3 ≤ D - 0,8872 x P.

Selon un mode de réalisation préféré, l'angle de conicité α° du tronc de cône défini par la crête du deuxième filetage 4 est de 9° (cet angle de conicité α° pouvant être plus généralement compris entre 1° et 60°).

Parallèlement, en référence à la figure 3, le ou les taraudages filetés 5 de la plaque 1 peuvent présenter une crête définissant un deuxième tronc de cône, dont l'angle de conicité B° est généralement inférieur à 60°.

Dans un mode de réalisation particulier, illustré sur la figure 4, l'angle de conicité B° du tronc de cône défini par le taraudage fileté 5 est égal à l'angle de conicité α° du tronc de cône défini par la crête du deuxième filetage 4. Il est toutefois envisageable que l'angle de conicité B° du tronc de cône défini par le taraudage fileté 5 soit égal à 0°.

Par ailleurs, tel qu'illustré par la figure 4, le premier filetage 3 de la tige filetée présente un pas noté PAS 1 et le deuxième filetage 4 de la tige filetée présente un pas noté PAS 2, avec PAS 1 ≥ PAS 2.

Selon un mode de réalisation préférentiel, le deuxième filetage est un filetage à deux filets. Ainsi, le pas apparent
$$PAS\ 2 = \frac{PAS\ 1.}{2}$$

Plus généralement, le deuxième filetage est un filetage à n filets, et
$$PAS\ 2 = \frac{PAS\ 1.}{n}$$

La tige filetée 2 présente classiquement un évidement 22 dont la section est prévue pour recevoir un outil de vissage de forme complémentaire.

Selon une autre caractéristique de l'invention, la tête de vissage 20 de la tige filetée présente un élément terminal 200 destiné à venir se loger intégralement dans la plaque 1 (tel qu'illustré par la figure 4), cet élément terminal présentant une surface destinée à venir en butée contre une surface 8 de forme complémentaire ménagée dans la plaque.

Cette surface de l'élément terminal 200, et celle de forme complémentaire ménagée dans la plaque présente des formes choisies en particulier parmi les formes suivantes :

- tronc de sphère ;
- tronc de cône ;
- annulaire et plate.

En référence à la figure 5, on décrit ci-après une tige filetée d'un dispositif d'ostéosynthèse selon une variante de l'invention.

Tel que cela apparaît sur cette figure, la tige filetée présente une tête de vissage 20 et, à son extrémité opposée à la tête de vissage, une base 21, la tige filetée présentant selon cette variante trois filetages successifs entre la base 21 et la tête de vissage 20.

Dans une portion de la tige s'étendant à partir de la tête 20, la tige filetée présente un filetage, dit deuxième filetage 4, destiné à coopérer avec un taraudage fileté ménagé dans la plaque. Ce deuxième filetage 4 s'étend, selon le principe général de l'invention, à partir d'un corps de forme cylindrique et présente un filet dont la crête définit un tronc de cône.

Sur une portion s'étendant à partir de la base 21 de la tige filetée, le premier filetage 3 destiné à assurer l'ancrage de la tige dans un os se décompose en deux parties 30, 31.

La première partie 30, dit partie d'extrémité, s'étend à partir de la base 21 et présente un filetage cylindrique.

La deuxième partie 31, dite partie intermédiaire, s'étend entre la première partie 30 et le deuxième filetage 4. Cette partie intermédiaire présente un filetage conique, dont l'angle de conicité est préférentiellement de 32°, et plus généralement compris entre 10° et 60°.

On note que cette partie intermédiaire est réalisée de façon à assurer une liaison entre la partie d'extrémité du premier filetage et le deuxième filetage. En d'autres termes, le plus petit diamètre de filet et le plus petit diamètre de fond de filet de la partie intermédiaire correspondent au diamètre de filet et au diamètre de fond de filet du filetage de la partie d'extrémité. De plus, le plus grand diamètre de filet et le plus grand diamètre de fond de filet de la partie intermédiaire correspondent au plus petit diamètre de filet et au plus petit diamètre de fond de filet du deuxième filetage 4.

## Revendications

1. Dispositif d'ostéosynthèse comprenant une plaque (1) destinée à être appliquée sur un os et au moins une tige (2) filetée destinée à assurer le maintien de la plaque sur l'os, ladite tige (2) présentant un premier filetage (3) destiné à assurer l'ancrage de ladite tige (2) dans un os et un deuxième filetage (4) destiné à coopérer avec un taraudage fileté (5) ménagé dans ladite plaque, le filet du premier filetage (3) présentant un premier pas, le deuxième filetage (4) présentant un deuxième pas inférieur ou égal audit premier pas, la crête dudit deuxième filetage (4) définissant un tronc de cône et ledit taraudage fileté (5) présentant une crête définissant un deuxième tronc de cône, **caractérisé en ce que** le fond de filet dudit deuxième filetage (4) définit un corps de forme cylindrique.

2. Dispositif d'ostéosynthèse selon la revendication 1, **caractérisé en ce que** ledit deuxième filetage (4) est obtenu par écrétage d'une vis présentant un diamètre nominal D et un pas P, le fond de filet cylindrique dudit deuxième filetage présentant un diamètre d3 tel que $d3 > D - 1,2268 \times P$.

3. Dispositif d'ostéosynthèse selon la revendication 2, **caractérisé en ce que** le diamètre de fond de filet d3 dudit deuxième filetage (4) est tel que $d3 \leq D - 0,8872 \times P$.

4. Dispositif d'ostéosynthèse selon l'une quelconque des revendications 1 à 3, **caractérisé en ce que** l'angle de conicité dudit tronc de cône est compris entre 1° et 60°.

5. Dispositif d'ostéosynthèse selon la revendication 4, **caractérisé en ce que** l'angle de conicité dudit tronc de cône est de 9°.

6. Dispositif d'ostéosynthèse selon l'une quelconque des revendications 1 à 5, **caractérisé en ce que** ladite tige (2) présente une tête (20) surmontant ledit deuxième filetage (4), ladite tête (20) étant apte à venir se loger complètement dans ladite plaque (1) et présentant une surface destinée à venir en butée contre une surface de forme complémentaire ménagée dans ladite plaque (1).

7. Dispositif d'ostéosynthèse selon la revendication 6, **caractérisé en ce que** ladite surface de ladite tête (20) et ladite surface de forme complémentaire ménagée dans ladite plaque (1) présentent une forme appartenant au groupe suivant :

   - tronc de sphère ;
   - tronc de cône ;
   - annulaire et plate.

8. Dispositif d'ostéosynthèse selon l'une quelconque des revendications 1 à 7, **caractérisé en ce que** ledit premier filetage (3) se décompose en deux parties :

   - une partie d'extrémité présentant un filetage cylindrique ;
   - une partie intermédiaire s'étendant entre ladite partie d'extrémité et ledit deuxième filetage, ladite partie intermédiaire présentant un filetage conique.

## Patentansprüche

1. Osteosynthesevorrichtung, umfassend eine Platte (1), die bestimmt ist, auf einem Knochen aufgesetzt zu sein, und mindestens einen Gewindestift (2), der bestimmt ist, den Halt der Platte auf dem Knochen sicherzustellen, wobei der Stift (2) ein erstes Gewinde (3), das bestimmt ist, die Verankerung des Stifts (2) in einem Knochen sicherzustellen, und ein zweites Gewinde (4), das bestimmt ist, mit einer gewindeten Bohrung (5), die in die Platte eingearbeitet ist, zusammenzuwirken, umfasst, wobei der Schraubengang des ersten Gewindes (3) eine erste Steigung aufweist, wobei das zweite Gewinde (4) eine zweite Steigung aufweist, die kleiner als oder gleich der ersten Steigung ist, wobei die Spitze des zweiten Gewindes (4) einen Kegelstumpf definiert und die gewindete Bohrung (5) eine Spitze aufweist, die einen zweiten Kegelstumpf definiert, **dadurch gekennzeichnet, dass** der Boden des Schraubengangs des zweiten Gewindes (4) einen zylinderförmigen Körper definiert.

2. Osteosynthesevorrichtung nach Anspruch 1, **dadurch gekennzeichnet, dass** das zweite Gewinde (4) durch Nivellierung einer Schraube hergestellt ist, die einen Nenndurchmesser D und eine Steigung P aufweist, wobei der zylindrische Schraubengangboden des zweiten Gewindes einen Durchmesser d3 aufweist, so dass $d3 > D - 1,2268 \times P$ ist.

3. Osteosynthesevorrichtung nach Anspruch 2, **dadurch gekennzeichnet, dass** der Durchmesser des Schraubengangbodens d3 des zweiten Gewindes (4) derart ist, dass $d3 \leq D - 0,8872 \times P$ ist.

**4.** Osteosynthesevorrichtung nach einem der Ansprüche 1 bis 3, **dadurch gekennzeichnet, dass** der Konizitätswinkel des Kegelstumpfs zwischen 1° und 60° inklusive ist.

**5.** Osteosynthesevorrichtung nach Anspruch 4, **dadurch gekennzeichnet, dass** der Konizitätswinkel des Kegelstumpfs 9° beträgt.

**6.** Osteosynthesevorrichtung nach einem der Ansprüche 1 bis 5, **dadurch gekennzeichnet, dass** der Stift (2) einen Kopf (20) über dem zweiten Gewinde (4) aufweist, wobei der Kopf (20) imstande ist, vollständig in der Platte (1) aufgenommen zu sein und eine Oberfläche aufweist, die bestimmt ist, an einer komplementär geformten Oberfläche anzuschlagen, die in der Platte (1) eingearbeitet ist.

**7.** Osteosynthesevorrichtung nach Anspruch 6, **dadurch gekennzeichnet, dass** die Oberfläche des Kopfs (20) und die komplementär geformte Oberfläche, die in der Platte (1) eingearbeitet ist, eine Form aufweisen, die zu der folgenden Gruppe gehört:

   - Kugelstumpf,
   - Kegelstumpf,
   - ringförmig und flach.

**8.** Osteosynthesevorrichtung nach einem der Ansprüche 1 bis 7, **dadurch gekennzeichnet, dass** das erste Gewinde (3) in zwei Teile zerfällt:

   - einen Endteil, der ein zylindrisches Gewinde aufweist,
   - einen Zwischenteil, der sich zwischen dem Endteil und dem zweiten Gewinde erstreckt, wobei der Zwischenteil ein konisches Gewinde aufweist.

**Claims**

**1.** An osteosynthesis device comprising a plate (1) intended to be applied on a bone and at least one threaded rod (2) intended to ensure the maintenance of the plate on the bone, said rod (2) having a first threading (3) intended to ensure the anchoring of said rod (2) in a bone and a second thread (4) intended to cooperate with a threaded tapping (5) arranged in said plate, the thread of the first threading (3) having a first pitch, the second threading (4) having a second pitch smaller than or equal to said first pitch, the crest of said second threading (4) defining a cone trunk and said threaded tapping (5) having a crest defining a second cone trunk, **characterized in that** the thread bottom of said second threading (4) defines a cylindrical body.

**2.** The osteosynthesis device according to claim 1, **characterized in that** said second threading (4) is obtained by trimming a screw having a nominal diameter D and a pitch P, the cylindrical thread bottom of said second threading having a diameter d3 such that d3 > D - 1.2268 X P.

**3.** The osteosynthesis device according to claim 2, **characterized in that** the thread bottom diameter d3 of said second threading (4) is such that $d3 \leq D - 0.8872 \times P$.

**4.** The osteosynthesis device according to any one of claims 1 to 3, **characterized in that** the conic angle of said cone trunk is comprised between 1° and 60°.

**5.** The osteosynthesis device according to claim 4, **characterized in that** the conic angle of said cone trunk is 9°.

**6.** The osteosynthesis device according to any one of claims 1 to 5, **characterized in that** said rod (2) has a head (20) topping said second threading (4), said head (20) being able to be housed completely in said plate (1) and having a surface intended to abut against a surface with a complementary shape arranged in said plate (1).

**7.** The osteosynthesis device according to claim 6, **characterized in that** said surface of said head (20) and said surface with a complementary shape arranged in said plate (1) have a shape belonging to the following group:

   - sphere trunk;
   - cone trunk;
   - annular and flat.

**8.** The osteosynthesis device according to any one of claims 1 to 7, **characterized in that** said first threading (3) is broken down into two parts:

   - an end part having a cylindrical threading;
   - an intermediate part extending between said end part and said second threading, said intermediate part having a conical threading.

<u>Fig. 1</u>

<u>Fig. 2</u>

<u>Fig. 3</u>

B°

EP 2 611 376 B1

Fig. 4

Fig. 4 bis

Fig. 5

**EP 2 611 376 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20080306550 A1